# EUROPÄISCHE PATENTSCHRIFT

(11) **EP 3 373 835 B1**
(45) Veröffentlichungstag und Bekanntmachung des Hinweises auf die Patenterteilung: **10.08.2022**
(21) Anmeldenummer: 16794517.9
(22) Anmeldetag: 09.11.2016
(51) Int. Cl.: A61B 34/30, A61F 2/30, B29C 67/00

(54) **ROBOTERSYSTEM ZUM IN-SITU IMPLANTATDRUCK**
ROBOT SYSTEM FOR IN SITU IMPLANT PRINTING
SYSTÈME ROBOTISÉ POUR IMPRESSION D'IMPLANT

(30) Priorität: 10.11.2015 DE 102015222117
(43) Veröffentlichungstag der Anmeldung: 19.09.2018
(73) Patentinhaber: KUKA Deutschland GmbH, 86165 Augsburg (DE)
(72) Erfinder: KEIBEL, Andreas, 86161 Augsburg (DE); REICHL, Tobias, 80939 München (DE); KELLER, Henrik, 86153 Augsburg (DE)
(74) Vertreter: Oelke, Jochen
(86) Internationale Anmeldenummer: PCT/EP2016/001855
(87) Internationale Veröffentlichungsnummer: WO 2017/080646

(56) Entgegenhaltungen:
- WO-A1-2015/054577
- WO-A2-2006/020685
- US-A1- 2003 100 824

## Beschreibung

### 1. Technischer Bereich

Die vorliegende Erfindung betrifft ein System zum Behandeln eines Patienten.

### 2. Technischer Hintergrund

Es gibt eine Vielzahl von Implantaten, welche als Ersatzmaterial oder Aufbaumaterial in einen menschlichen Körper eingesetzt werden. Ein Knochenimplantat, z. B., kann in einen Knochen eingesetzt werden, um beispielsweise einen Knochenbruch zu behandeln.

Seit einigen Jahren werden in der modernen Medizin Implantate mittels 3D-Druck hergestellt. Dabei werden entsprechende 3D-Computermodelle mittels eines 3D-Druckers in reale Bauteile umgesetzt. Der Aufbau des Implantats erfolgt computergesteuert aus einem oder mehreren flüssigen oder festen Werkstoffen, wobei physikalische oder chemische Härtungs- oder Schmelzprozesse stattfinden können. Die Werkstoffe, aus welchen die Implantate mittels 3D-Druck gedruckt werden, können beispielsweise Kunststoffe, Kunstharze, Keramiken oder Metalle umfassen.

Der 3D-Druck erlaubt es Implantate herzustellen, welche eine präzise an die jeweilige Anatomie angepasste Form aufweisen. Die Implantate können beispielweise im Krankenhaus oder von einem Lieferanten angefertigt werden, und anschließend während eines operativen Eingriffs in den Patienten eingesetzt werden.

Die WO2015/054577A1 offenbart einen 3D-Drucker zum Design, Herstellung und Aufbau von Objekten aus einem biologisch kompatiblen Material.

Die WO2006/020685A2 offenbart einen 3D-Drucker zur variablen Herstellung eines Objektes mittels austauschbarer Druckeinheiten.

Die US2003/0100824A1 offenbart einen 3D-Drucker zum gezielten Aufbringen eines Druckmaterials durch einstellbare Druckköpfe.

Es ist eine Aufgabe der vorliegenden Erfindung ein System bereitzustellen, mit welchem verbesserte Implantate hergestellt werden können. Diese und weitere Aufgaben, die aus der folgenden Beschreibung ersichtlich werden, werden durch den Gegenstand des Anspruchs 1 gelöst. Die Unteransprüche betreffen bevorzugte Ausführungsformen der Erfindung.

### 3. Inhalt der Erfindung

Die vorliegende Erfindung betrifft ein Robotersystem zum Behandeln eines Patienten. Das Robotersystem umfasst einen mehrachsigen Gelenkarmroboter sowie eine Druckvorrichtung, welche von dem Gelenkarmroboter geführt wird. Die Druckvorrichtung ist dabei zum Aufbauen eines Implantats durch Drucken eines biokompatiblen Druckmaterials eingerichtet.

Hierzu kann die Druckvorrichtung einen entsprechenden Druckkopf umfassen, mit welchem das Druckmaterial gezielt auf ein Substrat aufgebracht werden kann. Die Druckvorrichtung kann dabei für den Druck notwendige Hardware umfassen, wie beispielsweise ein Extrusionselement, Leitungen für das Druckmaterial, oder auch eine eigene Drucksteuerung. Nicht alle diese Elemente müssen an dem Gelenkarmroboter bereitgestellt sein, so kann eine Drucksteuerung beispielsweise separat von diesem implementiert sein.

Das Druckmaterial zeichnet sich dabei vorzugsweise durch eine gute Verträglichkeit aus, und eignet sich auch für einen längeren Einsatz des Implantats in dem Patienten. Das Druckmaterial kann dabei eine gewünschte Steifigkeit des Implantats ermöglichen.

Weiter ist eine Steuerung vorgesehen, welche eingerichtet ist, um den Gelenkarmroboter an dem Patient zu positionieren und das Implantat in dem Patienten aufzubauen. Die Steuerung ist dabei derart eingerichtet, um das Implantat in-situ in dem Patienten aufzubauen. Der Patient kann dabei ein Lebewesen, wie beispielsweise ein Mensch oder ein Tier sein. Die Druckvorrichtung kann insbesondere das Implantat mittels 3D-Druck aufbauen. Der Begriff Steuerung ist hierbei breit zu verstehen und die Steuerung kann viele, auch dezentral angeordnete Steuervorrichtungen, wie etwa Computer o.ä. umfassen. Insbesondere kann die Steuerung mehrere, unterschiedliche Steuervorrichtungen umfassen, etwa eine Steuervorrichtung zur Steuerung des Roboters (Robotersteuerung) und eine Steuervorrichtung zur Steuerung der Druckvorrichtung (Drucksteuerung). Alternativ kann die Steuerung auch nur aus einer einzigen geeigneten Steuervorrichtung bestehen.

Das erfindungsgemäße Robotersystem erlaubt somit ein in-situ-Drucken des Implantats direkt am Einsetzungsort des Implantats. Es kann somit ein Implantat direkt im Körper des Patienten aufgebaut werden, so dass dieses bestmöglich der Anatomie des Patienten angepasst sein kann. Ein Drucken des Implantats und folgendes Einsetzen als zweistufiger Prozess ist nicht mehr notwendig. Vielmehr kann das Implantat in-situ direkt an der betreffenden Stelle in dem Patienten gedruckt werden. Das Implantat kann dabei vorzugsweise einstückig gedruckt werden. Es können auch einzelne Elemente des Implantats in dem Patienten gedruckt werden, welche anschließend mit entsprechenden Verbindungsmitteln oder Kopplungen verbunden werden können.

Ein Gelenkarmroboter als Roboter kann eine universell einsetzbare, frei programmierbare Handhabungsvorrichtung sein. Die eigentliche Kinematik eines Roboters kann als Manipulator bezeichnet werden. Sie kann bei einem Gelenkarmroboter mehrere Glieder umfassen, welche beweglich zueinander eingerichtet sind. Hierzu können entsprechende Antriebe, und insbesondere Servomotoren bereitgestellt sein, welche die einzelnen Glieder beispielsweise elektrisch, hydraulisch oder pneumatisch bewegen können. Die Regelung der Antriebe kann mittels der Steuerung erfolgen, welche eine Roboterbewegung entsprechend einer vorprogrammierten Bahnplanung umsetzen kann. Der Gelenkarmroboter kann ortsfest installiert sein, oder am Patienten bereitgestellt sein. Beispielsweise kann der Gelenkarmroboter auf dem Patienten stehen, und/oder durch diesen gestützt werden. Somit wird ein nahezu konstantes räumliches Verhältnis zwischen dem Gelenkarmroboter und dem Patienten ermöglicht.

Der mehrachsige Gelenkarmroboter weist vorzugsweise zumindest sechs Freiheitsgrade in seiner Bewegung auf. Er ist somit sehr flexibel und kann eine geeignete Pose einnehmen, um das Implantat in dem Patienten zu drucken bzw. aufzubauen. Es kann auch ein flexibler Manipulatorarm oder sogenannter kontinuierlicher Manipulatorarm verwendet werden, welcher eine hohe Anzahl von Freiheitsgraden aufweist. Ein solcher flexibler Manipulatorarm kann mit einem herkömmlichen mehrachsigen Gelenkarmroboter kombiniert werden, um eine gewünschte Flexibilität des Systems zu ermöglichen.

Der mehrachsige Gelenkarmroboter umfasst Sensoren, welche eingerichtet sind, um Kräfte und/oder Drehmomente zu erfassen, die auf den Gelenkarmroboter wirken. Diese Sensoren können als Kraft-Moment-Sensoren ausgebildet sein, und beispielsweise mittels Dehnungsmessstreifen Kräfte bzw. Drehmomente detektieren. Somit können Wechselwirkungen zwischen dem Gelenkarmroboter und seiner Umgebung präzise erfasst und ausgewertet werden, sodass beispielsweise ungewollte Verletzungen des Patienten oder Beschädigungen des Gelenkarmroboters vermieden werden können. Ferner kann der Gelenkarmroboter in einem haptischen Modus arbeiten, und somit beispielsweise genau den Druckort des Implantats erkennen.

Der haptische Modus kann auch genutzt werden, um den Roboter manuell zu führen, beispielsweise per Hand. D.h. die Kräfte und/oder Drehmomente werden durch einen Benutzer erzeugt, beispielsweise durch dessen Hand, und führen zu einer Reaktion des Gelenkarmroboters, beispielsweise ein Nachgeben in Kraft-/Momentenrichtung. Hierzu ist die Steuerung eingerichtet, den Gelenkarmroboter basierend auf einer Führung des Gelenkarmroboters per Hand zu positionieren. Der Gelenkarmroboter kann in diesem Modus mittels haptischer Ausgaben den Benutzer bei der Anwendung unterstützen. Solche Ausgaben können beispielhaft virtuelle Hindernisse darstellen, die den Arbeitsbereich des Roboters einschränken, um die Sicherheit oder Genauigkeit zu erhöhen oder auch einen Eingriff zu erleichtern oder zu trainieren. Weitere Beispiele für haptische Ausgaben können Vibrationen oder veränderliche Steifigkeit des Roboters sein.

Vorzugsweise ist zwischen einem Handflansch des Gelenkarmroboters und der Druckvorrichtung eine Linearachse vorgesehen. Diese Linearachse kann dabei eine lineare Bewegung eines Druckkopfes ermöglichen, sodass beispielsweise eine Eindringtiefe des Druckkopfes der Druckvorrichtung in den Patienten präzise gesteuert und kontrolliert werden kann. Es können auch anders ausgestaltete Antriebe oder Glieder zwischen dem Handflansch und der Druckvorrichtung bereitgestellt sein, um weitere Freiheitsgrade zu ermöglichen.

Vorzugsweise umfasst die Druckvorrichtung eine steuerbare und/oder eine drehbare gekrümmte Nadel. Somit kann eine bestimmte Trajektorie beim Eindringen in den Patienten präzise verfolgt werden. Insbesondere vorzugsweise ist eine Führung der Druckvorrichtung als eine solche steuerbare Nadel und/oder eine solche drehbare gekrümmte Nadel ausgestaltet. Die Nadeln können gekrümmte elastische Röhrchen umfassen, die übereinander geschoben sein können. Durch ein individuelles Drehen jedes der Röhrchen, vorzugsweise mittels eines entsprechenden Antriebs, kann eine gewünschte Trajektorie beim Vorschub in Weichgewebe erreicht werden. Somit kann ein präzises und minimalinvasives Erreichen des Druckortes und Drucken des Implantats in dem Patienten gewährleistet werden.

Vorzugsweise sind an dem mehrachsigen Gelenkarmroboter weiterhin Mittel zum Aushärten des biokompatiblen Druckmaterials bereitgestellt. Wenn das Druckmaterial beispielsweise ein UV-härtendes Material ist, kann das Mittel zum Aushärten insbesondere derart eingerichtet sein, um UV-Strahlung bereitzustellen bzw. das Druckmaterial mittels UV-Strahlung auszuhärten. Somit kann mittels des Robotersystems minimalinvasiv das Implantat aufgebaut werden, da keine zusätzlichen Schnitte oder Zugangskanäle notwendig sind, um das Implantat aufzubauen.

Vorzugsweise ist die Steuerung eingerichtet, um das Implantat zumindest teilweise um eine anatomische Struktur und/oder zumindest teilweise in einem anatomischen Hohlraum in dem Patient aufzubauen. Das vorzugsweise einstückig aufgebaute Implantat kann somit im Körper des Patienten anatomische Strukturen umschließen, wodurch sich unter anderem ein besserer Halt des Implantats im Patienten ergibt. Verschraubungen sind hierzu nicht notwendig, alleine durch die Passform des Implantats am Druckort im Patienten kann ein sicherer Halt gewährleistet werden. Durch das Füllen von anatomischen Hohlräumen mittels des Druckmaterials bzw. des Implantats kann neben einer präzisen Passform mit verbessertem Halt ferner eine verbesserte Verwachsung des Implantats erzielt werden.

Weiter betrifft die vorliegende Offenbarung ein nicht beanspruchtes Verfahren zum Behandeln eines Patienten mittels eines mehrachsigen Gelenkarmroboters. Der Gelenkarmroboter führt dabei eine Druckvorrichtung, welche zum Aufbauen eines Implantats durch Drucken eines biokompatiblen Druckmaterials eingerichtet ist. Das Verfahren umfasst ein Ansteuern des Gelenkarmroboters, um die Druckvorrichtung an dem Patienten zu positionieren, und ein Aufbauen des Implantats in dem Patienten mittels der Druckvorrichtung. Durch die Verwendung des Gelenkarmroboters kann eine hohe Genauigkeit erreicht werden. Vorzugsweise wird das Implantat dabei in-situ in dem Patienten aufgebaut. Der Fachmann versteht, dass das Verfahren zum Behandeln eines Patienten mittels des oben-beschriebenen Robotersystems erfolgen kann.

Vorzugsweise erfolgen vor dem Aufbauen des Implantats ferner ein Bereitstellen von Gewebe-Bilddaten einer zu behandelnden Stelle des Patienten, und ein Erstellen einer Bahnplanung unter Berücksichtigung der bereitgestellten Gewebe-Bilddaten. Die Gewebe-Bilddaten können Magnetresonanztomographie-(MRT-) oder Computertomographie- (CT-) Daten umfassen. Prinzipiell können auch weitere Bildgebungsverfahren eingesetzt werden, um Gewebe-Bilddaten aufzuzeichnen. Die zu behandelnde Stelle kann die Stelle an oder in dem Patienten darstellen, an welcher das Implantat aufgebaut werden soll. Die Bahnplanung kann die zumindest teilweise automatisch durchzuführenden Bewegungen und Aktionen des Gelenkarmroboters und/oder der Druckvorrichtung beschreiben. Das Erstellen der Bahnplanung kann dabei ein Simulieren des Druckprozesses umfassen, welches rechnergestützt erfolgen kann. Somit kann der Aufbau des Implantats in dem Patienten präzise geplant werden, ausgehend von den Gewebe-Bilddaten. Ein Situs kann dazu datentechnisch segmentiert werden, und das Erstellen der Bahnplanung kann interaktiv mit einem Bediener erfolgen.

Insbesondere vorzugsweise erfolgt ferner vor dem Aufbauen des Implantats ein Bereitstellen von Implantat-Strukturdaten des Implantats, und ein Vergleichen der Implantat-Strukturdaten mit den bereitgestellten Gewebe-Bilddaten. Die Implantat-Strukturdaten können dabei auf einer entsprechenden Datenbank basieren, welche beispielsweise CAD-Daten des Implantats umfasst. Es kann somit eine virtuelle Begutachtung und Anpassung des in-situ zu druckenden Implantats durchgeführt werden.

Vorzugsweise ist das Implantat zumindest teilweise um eine anatomische Struktur und/oder teilweise in einem anatomischen Hohlraum in dem Patienten aufgebaut. Hierdurch kann ein präziser Halt des Implantats gewährleistet werden.

Vorzugsweise umfasst das nicht beanspruchte Verfahren ferner ein Detektieren einer Führung des Gelenkarmroboters per Hand durch Erfassen von Kräften und/oder Drehmomenten, die auf den Gelenkarmroboter infolge der Führung wirken, und ein Ansteuern des Gelenkarmroboters (2), um der Führung per Hand zu folgen. Somit kann ein Bediener den Gelenkarmroboter intuitiv steuern, beispielsweise während oder zur Vorbereitung des Druckprozesses.

Das biokompatible Druckmaterial kann dabei ein UV-härtendes und/oder ein wasser-härtendes Material sein. Das biokompatible Druckmaterial ruft dabei keine Implikationen mit dem Gewebe des Patienten hervor. So kann ein thermisch unkritisches viskoses Substrat in-situ per UV-Licht gehärtet werden. Es kann ferner ein Substrat eingebracht werden, welches durch den hohen Feuchtigkeitsgehalt der Umgebung schnell aushärtet.

Vorzugsweise ist die Druckvorrichtung zum Aufbauen des Implantats mittels eines Robocasting-Prozesses oder mittels Schmelzschichtung eingerichtet. Somit kann abhängig von dem jeweiligen Eingriff ein geeignetes Verfahren verwendet werden.

Vorzugsweise ist das Implantat als intrakorporale Stützkonstruktion ausgestaltet, welche beispielsweise eine Knochenbrücke stabilisieren kann. Eine externe Schiene ist nicht notwendig.

Vorzugsweise ist das Implantat als Stützstruktur ausgestaltet, insbesondere für weiches Gewebe. Somit können beispielsweise Netze um Blutgefäße gedruckt werden, um Aneurysmen vorzubeugen oder zurückzudrängen.

Vorzugsweise ist das Implantat als künstliches Stützgewebe ausgestaltet, auf welches sich beispielsweise neue Zellen eines gewünschten Typs ansiedeln können.

Vorzugsweise ist das Implantat als Prothese ausgestaltet, so dass eine Prothese direkt am anatomischen Ort ihres Einsatzes, wie beispielsweise an einem Knie, einer Bandscheibe, einem Wirbel, etc. aufgebaut bzw. gedruckt werden kann.

Vorzugsweise ist das Implantat als Knochenersatz ausgestaltet, so dass die Tragfähigkeit eines Knochens zumindest teilweise wiederhergestellt werden kann. Ein unstrukturiertes Einspritzen von Zement mittels Kyphoplastie ist nicht notwendig, da das in-situ aufgebaute Implantat passend zu den vorhanden Strukturen modelliert werden kann.

Vorzugsweise ist das Implantat als Veneer ausgestaltet, welches im Dentalbereich direkt auf Zähne aufgebracht bzw. gedruckt werden kann.

Vorzugsweise ist das Implantat als modelliertes Substrat ausgestaltet, welches beispielsweise in der plastischen Chirurgie eingesetzt werden kann. Somit kann durch die modellierte Einbringung von Substraten ästhetische Oberflächen strukturiert werden, die gegebenenfalls durch Traumata verloren gegangen sein können.

Die Verwendung eines mehrachsigen Gelenkarmroboters mit einer geführten Druckvorrichtung zum in-situ Aufbauen eines Implantats ermöglicht vielfältige Einsatzmöglichkeiten. Aufgrund der Flexibilität und Universalität des Systems können verschiedenartige Implantate mit einem erfindungsgemäßen Robotersystem aufgebaut werden. Umständliche Modifikationen des Systems sind dazu vorteilhaft nicht notwendig.

Das nicht beanspruchte Verfahren zum Behandeln eines Patienten ist vorteilhaft minimalinvasiv, da kein großer Schnitt oder ein aufwendiger Zugangskanal erforderlich ist, um ein zuvor gedrucktes Implantat in den Patienten einzubringen. Insbesondere größere Implantate können somit vorteilhaft direkt in-situ in dem Patienten gedruckt werden. Die Verwendung des mehrachsigen Gelenkarmroboters ermöglicht dabei eine hohe Genauigkeit des Implantataufbaus.

### 4. Ausführungsbeispiele

Im Folgenden wird die vorliegende Erfindung unter Bezugnahme auf die beiliegende Figur näher beschrieben. Es zeigt:

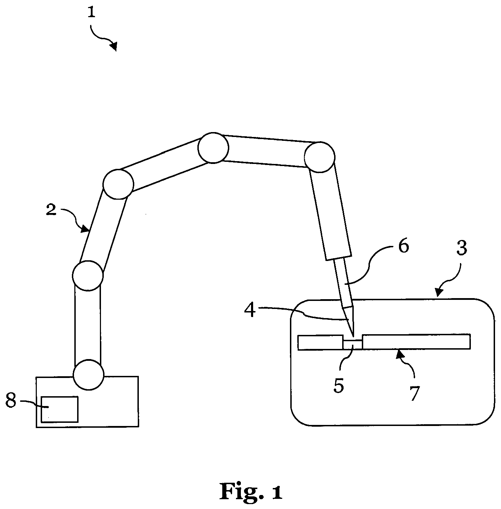

Fig. 1 ein Robotersystem zum Behandeln eines Patienten gemäß einer Ausführungsform.

In der Figur 1 ist ein Robotersystem 1 dargestellt. Dieses umfasst einen mehrachsigen Gelenkarmroboter 2, welcher mehrere Achsen und Glieder umfasst, die beweglich zueinander eingerichtet sind. Die Bewegung des Gelenkarmroboters 2 wird mittels einer entsprechenden Steuerung 8 geregelt, die an dem Gelenkarmroboter 2 oder separat zu diesem bereitgestellt sein kann. Der Gelenkarmroboter 2 ist ortsfest über einen Roboterfuß mit der Umgebung verbunden, kann aber auch ortsflexibel sein. Der Gelenkarmroboter 2 führt als Endeffektor eine Druckvorrichtung mit einem Druckkopf 4, welche zum Aufbauen eines Implantats 5 durch Drucken eines biokompatiblen Druckmaterials eingerichtet ist. Hierzu kann der Manipulator 2 eine entsprechende Bevorratung für das biokompatible Druckmaterial mit sich führen.

Der Druckkopf 4 ist über eine Linearachse 6 mit einem Handflansch des Gelenkarmroboters 2 verbunden. Somit kann die Eindringtiefe des Druckkopfes 4 in einen Patienten 3 präzise gesteuert werden.

In der in Figur 1 dargestellten Situation wird mittels des mehrachsigen Gelenkarmroboters 2 ein Implantat 5 in einem Patienten 3 aufgebaut. Der beispielhafte Patient 3 ist hier nur schemenhaft dargestellt, und umfasst einen gebrochenen Knochen 7. Die Bruchstelle bildet die zu behandelnde Stelle. An dieser Bruchstelle bzw. in einer Lücke des Knochens 7 wird das Implantat 5 aufgebaut. Hierzu wird durch den Gelenkarmroboter 2 das biokompatible Druckmaterial an die entsprechende Stelle mittels des Druckkopfes aufgedruckt. Anschließend härtet das Druckmaterial aus, beispielsweise unter Einwirkung von UV-Strahlung, um ein belastbares Implantat 5 zu bilden.

Der Gelenkarmroboter 2 kann den Druckkopf 4 entweder teilautomatisch oder automatisch führen, oder der Gelenkarmroboter 2 kann teleoperiert geführt werden. Die Telerobotik ist ein Teilgebiet der Robotik, welche die Steuerung von Robotern aus der Distanz beschreibt. Der Gelenkarmroboter 2 kann einzelne Fertigungsabschnitte oder den gesamten Prozess durchführen.

Mit Hilfe einer bildgebungsgestützten Planungssoftware kann der in-situ-Implantataufbau genau geplant werden. Ausgehend von 2D- oder 3D-Computertomographiedaten oder 3D-Magnetresonanztomographiedaten kann der Situs innerhalb der Planungssoftware datentechnisch segmentiert, und der Herstellungs- bzw. Aufbauprozess interaktiv gestaltet werden. Es kann dabei insbesondere eine virtuelle Begutachtung ermöglicht werden, mit welcher eine 3D-Darstellung des Herstellungsprozesses bzw. des Implantataufbaus simuliert werden kann.

Ferner kann das fertige Implantat vorherberechnet werden. Mittels der Planungssoftware können die Originalbilddaten (z.B. CT- oder MRT-Bilddaten) vom Patienten mit dem zu generierenden Implantat die lagerichtig augmentiert werden. Sobald die Planung zufriedenstellend abgeschlossen ist, kann der tatsächliche Eingriff und Implantataufbau in-situ erfolgen.

Hierzu wird der Druckkopf 4 entsprechend der Planung in eine Druckposition in dem Patienten 3 geführt, und der Situs entsprechend der Aufgabenstellung vorbereitet. Hierzu kann Gas eingebracht werden, oder auch das Gewebe ausgerichtet werden. Ferner kann gegebenenfalls die Druckbasis freigelegt werden, auf welche das Implantat aufgedruckt werden soll. Die präzise Positionierung des Druckkopfes 4 erfolgt mittels des Gelenkarmroboters 2 und insbesondere der Linearachse 6, wodurch die Lage des Druckkopfes 4 genau gesteuert werden kann und diese somit präzise ausgerichtet werden kann. Der Aufbau des Implantats 5 kann dann entsprechend den Planungsvorgaben erfolgen.

Dabei kann ein Therapeut bzw. Arzt den Gelenkarmroboter 2 eigenständig führen, und die Prozessgrößen gegebenenfalls selber einstellen. Durch Hand-Auge-Koordination, z.B. mittels eines Endoskops, kann der Implantataufbau qualitativ beurteilt werden und gegebenenfalls angepasst werden. Die eigenständige Führung des Gelenkarmroboters 2 kann dabei auch mittels Roboter-Teleoperation erfolgen. Der Arzt kann sich dabei in einem anderen Zimmer befinden, und über eine entsprechende Konsole das System steuern. Das Teleoperationssystem kann hierzu vorzugsweise eine haptische Rückkopplung ermöglichen, sodass durch virtuell haptisch spürbare Widerstände ein Abweichen von einer geplanten Bahn oder Verletzung von angrenzendem Gewebe signalisiert und verhindert werden kann.

Der Gelenkarmroboter 2 kann auch vollautomatisch eigenständig positioniert werden, und den Implantataufbau autonom durchführen. Insbesondere kann das System 1 auch Prozessgrößen eigenständig führen, wie beispielsweise Druckraten oder Aushärtungsraten.

An den Achsen oder Gelenken des Gelenkarmroboters 2 bereitgestellte Kraft-Moment-Sensorik kann eine Kraftregelung des Gelenkarmroboters 2 ermöglichen. Die Kraftregelung kann dabei als Impedanz- oder Nachgiebigkeitsregelung ausgestaltet sein. Hierzu können die auf den Roboter 2 wirkenden Kräfte erfasst und analysiert werden, sodass auf äußere Einflüsse reagiert werden kann, um beispielsweise Kontaktkräfte zwischen dem Druckkopf 4 und dem Patienten 3 zu reduzieren. Somit kann insbesondere eine mögliche Patientenbewegung erfasst und entsprechend reagiert werden, um mögliche Verletzungen zu vermeiden. Hierzu kann insbesondere weitere Sensorik direkt am Druckkopf 4 bereitgestellt sein, welche mit einer hohen Genauigkeit die Position und Bewegung des Druckkopfes 4 im Patienten 3 überwacht.

Der Fachmann versteht, dass die in der Figur 1 dargestellte Ausführungsform nur beispielhaft und nicht beschränkend ist. Die oben beschriebenen Merkmale müssen dabei nicht auf diese Ausführungsform beschränkt sein, sondern können auch Elemente weiterer Ausführungsformen im Sinne der vorliegenden Erfindung bilden.

### Bezugszeichenliste:

- 1: Robotersystem
- 2: Gelenkarmroboter
- 3: Patient
- 4: Druckkopf
- 5: Implantat
- 6: Linearachse
- 7: Knochen
- 8: Steuerung

## Patentansprüche

1. Robotersystem (1) zum Behandeln eines Patienten (3), umfassend:
einen mehrachsigen Gelenkarmroboter (2);
eine Druckvorrichtung (4), welche von dem Gelenkarmroboter (2) geführt wird und zum Aufbauen eines Implantats (5) durch Drucken eines biokompatiblen Druckmaterials eingerichtet ist; und
eine Steuerung (8), die eingerichtet ist, um den Gelenkarmroboter (2) an dem Patienten (3) zu positionieren und das Implantat (5) in dem Patienten (3) aufzubauen
**dadurch gekennzeichnet,**
**dass** der mehrachsige Gelenkarmroboter (2) Sensoren umfasst, die eingerichtet sind, um auf den Gelenkarmroboter (2) wirkende Kräfte und/oder Drehmomente zu erfassen und
**dass** die Steuerung (8) eingerichtet ist, den Gelenkarmroboter (2) basierend auf einer Führung des Gelenkarmroboters (2) per Hand zu positionieren.

2. Robotersystem (1) nach Anspruch 1, wobei der mehrachsige Gelenkarmroboter (2) zumindest 6 Freiheitsgrade in seiner Bewegung aufweist.

3. Robotersystem (1) nach einem der Ansprüche 1 oder 2, wobei zwischen einem Handflansch des Gelenkarmroboters (2) und der Druckvorrichtung (4) eine Linearachse (6) vorgesehen ist.

4. Robotersystem (1) nach einem der Ansprüche 1 bis 3, wobei die Druckvorrichtung (4) eine steuerbare und/oder drehbare gekrümmte Nadel umfasst, und wobei vorzugsweise eine Führung der Druckvorrichtung (4) als steuerbare und/oder drehbare gekrümmte Nadel ausgestaltet ist.

5. Robotersystem (1) nach einem der Ansprüche 1 bis 4, wobei an dem mehrachsigen Gelenkarmroboter (2) ferner Mittel zum Aushärten des biokompatiblen Druckmaterials bereitgestellt sind, und wobei die Mittel vorzugsweise eingerichtet sind, um das Druckmaterial mittels UV-Strahlung auszuhärten.

6. Robotersystem (1) nach einem der Ansprüche 1 bis 5, wobei die Steuerung (8) eingerichtet ist, um das Implantat (5) zumindest teilweise um eine anatomische Struktur und/oder zumindest teilweise in einem anatomischen Hohlraum in dem Patienten (3) aufzubauen.

7. Robotersystem (1) nach einem der Ansprüche 1 bis 6, wobei die Steuerung (8) eingerichtet ist, um das Implantat (5) in-situ in dem Patienten (3) aufzubauen.

8. Robotersystem (1) nach einem der Ansprüche 1 bis 7, wobei das biokompatible Druckmaterial ein UV-härtendes und/oder ein Wasser-härtendes Material ist.

9. Robotersystem (1) nach einem der Ansprüche 1 bis 8, wobei die Druckvorrichtung (4) zum Aufbauen des Implantats mittels eines Robocasting-Prozesses oder mittels Schmelzschichtung eingerichtet ist.

10. Robotersystem (1) nach einem der Ansprüche 1 bis 9, wobei das Implantat (5) als intrakorporale Stützkonstruktion, als Stützstruktur für weiches Gewebe, als künstliches Stützgewebe, als Prothese, als Knochenersatz, oder als Veneer oder als modelliertes Substrat ausgestaltet ist.

## Claims

1. Robot system (1) for treating a patient (3), comprising:
a multi-axis articulated arm robot (2);
a printing device (4), which is guided by the articulated arm robot (2) and is configured for constructing an implant (5) by printing a biocompatible print material; and
a controller (8), which is configured for positioning the articulated arm robot (2) on the patient (3) and for constructing the implant (5) in the patient (3),
**characterized in that**
the multi-axis articulated arm robot (2) comprises sensors, which are configured for detecting forces and/or torques acting on the articulated arm robot (2), and
the controller (8) is configured for positioning the articulated arm robot (2) based on a guidance of the articulated arm robot (2) by hand.

2. Robot system (1) according to Claim 1, wherein the multi-axis articulated arm robot (2) has at least 6 degrees of freedom in its movement.

3. Robot system (1) according to either of Claims 1 and 2, wherein a linear axle (6) is provided between a hand flange of the articulated arm robot (2) and the printing device (4).

4. Robot system (1) according to one of Claims 1 to 3, wherein the printing device (4) comprises a controllable and/or rotatable curved needle, and wherein preferably a guide of the printing device (4) is designed as a controllable and/or rotatable curved needle.

5. Robot system (1) according to one of Claims 1 to 4, wherein means for curing the biocompatible printing material are moreover made available on the multi-axis articulated arm robot (2), and wherein the means are preferably configured to cure the printing material by means of UV radiation.

6. Robot system (1) according to one of Claims 1 to 5, wherein the controller (8) is configured to construct the implant (5) at least partially around an anatomical structure and/or at least partially in an anatomical cavity in the patient (3).

7. Robot system (1) according to one of Claims 1 to 6, wherein the controller (8) is configured to construct the implant (5) in situ in the patient (3) .

8. Robot system (1) according to one of Claims 1 to 7, wherein the biocompatible printing material is a UV-curable and/or a water-curable material.

9. Robot system (1) according to one of Claims 1 to 8, wherein the printing device (4) is configured for constructing the implant by means of a robocasting process or by means of melt layering.

10. Robot system (1) according to one of Claims 1 to 9, wherein the implant (5) is designed as an intracorporeal support structure, as a support structure for soft tissue, as artificial support tissue, as a prosthesis, as bone replacement, or as a veneer or as a modelled substrate.

## Revendications

1. Système robotisé (1) destiné à traiter un patient (3), ledit système comprenant :
un robot à bras articulé multiaxes (2) ;
un dispositif d'impression (4) qui est guidé par le robot à bras articulé (2) et qui est conçu pour construire un implant (5) par impression d'un matériau d'impression biocompatible ; et
une commande (8) qui est conçue pour positionner le robot à bras articulé (2) sur le patient (3) et pour confectionner l'implant (5) dans le patient (3).
**caractérisé en ce que**
le robot à bras articulé multiaxes (2) comprend des capteurs qui sont conçus pour détecter des forces et/ou des couples agissant sur le robot à bras articulé (2) et
la commande (8) est conçue pour positionner le robot à bras articulé (2) sur la base d'un guidage du robot à bras articulé (2) à la main.

2. Système robotisé (1) selon la revendication 1, le robot à bras articulé multiaxes (2) comportant au moins 6 degrés de liberté dans son mouvement.

3. Système robotisé (1) selon l'une des revendications 1 ou 2, un axe linéaire (6) étant prévu entre une bride de main du robot à bras articulé (2) et le dispositif d'impression (4).

4. Système robotisé (1) selon l'une des revendications 1 à 3, le dispositif d'impression (4) comprenant une aiguille incurvée commandable et/ou rotative, et de préférence un guide du dispositif d'impression (4) étant conçu comme une aiguille incurvée commandable et/ou rotative.

5. Système robotisé (1) selon l'une des revendications 1 à 4, des moyens de durcissement du matériau d'impression biocompatible étant également prévus sur le robot à bras articulé multiaxes (2), et les moyens étant de préférence conçus pour durcir le matériau d'impression au moyen d'un rayonnement UV.

6. Système robotisé (1) selon l'une des revendications 1 à 5, la commande (8) étant conçue pour confectionner l'implant (5) au moins partiellement autour d'une structure anatomique et/ou au moins partiellement dans une cavité anatomique dans le patient (3).

7. Système robotisé (1) selon l'une des revendications 1 à 6, la commande (8) étant conçue pour confectionner l'implant (5) in situ dans le patient (3) .

8. Système robotisé (1) selon l'une des revendications 1 à 7, le matériau d'impression biocompatible étant un matériau durcissant aux UV et/ou à l'eau.

9. Système robotisé (1) selon l'une des revendications 1 à 8, le dispositif d'impression (4) étant conçu pour confectionner l'implant au moyen d'un procédé de robocasting ou par empilage de couches à l'état fondu.

10. Système robotisé (1) selon l'une des revendications 1 à 9, l'implant (5) étant conçu comme une structure de support intracorporelle, comme une structure de support de tissus mous, comme un tissu de support artificiel, comme une prothèse, comme un substitut osseux, ou comme une facette dentaire ou comme un substrat modelé.
